# EUROPEAN PATENT APPLICATION

(11) **EP 4 529 837 A1**
(43) Date of publication of application: **02.04.2025**
(21) Application number: 23199628.1
(22) Date of filing: 26.09.2023
(51) Int. Cl.: A61B 5/145, A61B 5/1455

(54) **COLORIMETRIC TEMPORARY TATTOO SENSORS**

(71) Applicant: Technische Universität Graz, 8010 Graz (AT)
(72) Inventor: Unger, Katrin, 8042 Graz (AT); Coclite, Anna Maria, 8010 Graz (AT)
(74) Representative: Patentanwaltskanzlei Matschnig & Forsthuber OG

(57) **Abstract**

The present invention relates to a temporary tattoo sensor for application to an individual's skin and for detecting physiological analytes within sweat, wherein the sensor comprises: a substrate layer configured to be placed on the skin and having a first side facing away from the skin and a second side facing the skin; a colorimetric layer disposed on the second side of the substrate layer; wherein the colorimetric layer comprises a hydrogel matrix and at least one indicator dye; wherein the hydrogel matrix is configured to accumulate sweat that segregates from the individual's skin and to act as a carrier matrix for immobilizing the indicator dye; and wherein the indicator dye is configured to visibly change color upon contact with at least one physiological analyte present in the sweat accumulated in the hydrogel. The invention further relates to methods for manufacturing a sweat sensor according to the invention and to methods of monitoring the health status of an individual by using the sweat sensor according to the invention.

## Description

### Field of the Invention

The invention relates to a sweat sensor for application to an individual's skin and for detecting physiological analytes within sweat of said individual. The invention further relates to methods for manufacturing a sweat sensor according to the invention and to methods of monitoring the health status of an individual by using the sweat sensor according to the invention.

### Background

The four main physiological fluids accessible from the outside of the body are sweat, saliva, tears, and urine which contain various chemical substances that provide insight about the state of health. Sweat is of particular interest as it is available via the complete skin surface, readily available and its composition can be directly linked to the biomarkers of blood, which is one of the "golden standards" in health monitoring. Sweat is a complex mixture of several ion species, ammonium, glucose, and lactate among others, and provides rich source of information about the physiological state of the body such as dehydration, anaerobic catabolism, stress hyperglycemia (diabetes shock), kidney failure or inebriation.

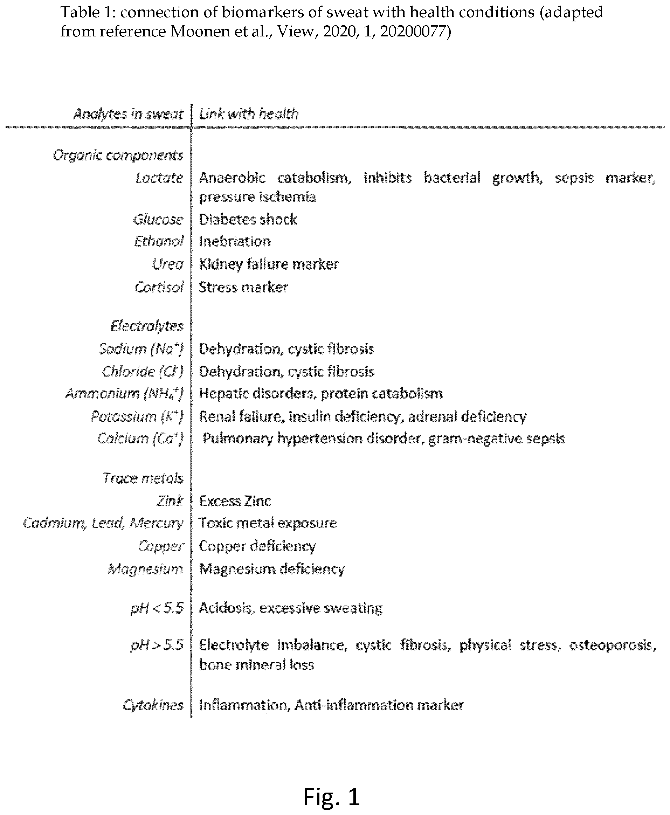

Smart wearable sensors are an effective method in health care diagnostics to detect health deterioration in early stage of diseases. Analyzing sweat is of particular interest, as it can be accessed without penetration of the skin, is readily available and its composition of biomarkers is linked to that of blood. An overview of wearable sweat sensor systems and the link of common components of sweat with health/disease conditions is given in a review Moonen et al [Moonen et al, View, 2020, 1, 20200077]. Fig. 1 of the figures appended to this disclosure shows in Table 1 the connection of biomarkers of sweat with health conditions, adapted from reference Moonen et al, View, 2020, 1, 20200077.

A fully integrated wearable sweat sensor typically consists of a sweat collecting part and a sensing part. Sweat collection can be divided into three approaches: (i) microfluidic systems using capillary forces to transport sweat through channel, (ii) absorption by wicking the sweat into the device and (iii) direct collection on the skin utilizing either a cavity or the topography of the skin itself for the collection.
**(i) Microfluidic systems** are widely used in wearable sweat sensors. Via capillary forces the fluid can be transported in channels with a volume precision down to nanoliter. The design of the microfluidic architecture can be straightforward and suitable for scale-up fabrication techniques (soft lithography or molding). Nevertheless, the volume between the sweat gland and the skin surface ranges within 10 µl - 20 µl. Typical sweat production ranges from 0.02 - 0.3 nl/min/gland for sedentary patients to 1 - 20 nl/min/gland for an average adult at rest, only during exercise it rises several orders of magnitude. A meager sweat rate hinders a fast detection with a typical response time of 10 min - 20 min. During this time diffusion of analytes through the channels can be expected resulting in a general delayed and less accurate time resolved measurement. Hence, microfluidic-based sweat collection is mainly applicable during physical exercise, where an elevated sweat rate can be expected.
**(ii) Absorption collection** is based on a material which is wicking sweat from the skin into the device. Compared to microfluidic methods, absorption techniques can be also applied at little sweat rate (sedentary patients) or when fast sweat collection is required. A disadvantage of this method is that the absorption pad is spacious which provides the possibility for the analytes to diffuse throughout the area. Consequently, the measurements have a poor time resolution.
(iii) **A direct on-skin collection** does not collect the sample and sequentially transport it to the sensor, but instead the sensor is placed directly on the skin. Sweat can be either collected in a small reservoir or directly within the small groves of the epidermis. The main advantage of this technique is, that no specific collection mechanism needs to be incorporated, which simplifies the fabrication process. However, this direct approach may cause interference of movement of the skin with the measurement. Additional, direct on-skin devices with small reservoirs often suppress removal of old sweat which results in a poor temporal resolution. Temporary tattoo-based sweat sensor devices fall into the category of direct on-skin collecting methods. Temporary tattoo-based sweat sensor devices known in the art comprise a sub micrometer thick tattoo layer being in intimate contact with the skin and acting as a carrier platform for flexible electrodes, which are in direct contact with sweat that is collected within the groves of the epidermis. The benefits of direct on-skin collection within tattoo-based approaches are the ultra-conformal adaption to the rough surface of epidermis, therefore minimizing the dead volume between the sample and the sensor, the deformable nature of tattoo demonstrating skin-alike properties and the water-vapor transmission through the sensor, providing the possibility for old sweat to evaporate. On the contrary, deformation of the sensor may cause electric signal artifacts, which needs to be considered.

As mentioned above, a fully integrated sweat sensor device consists of a collecting strategy and a sensor. Generally, typical detection strategies of the sensor imply electroanalytical or optical techniques. While the first mentioned strategy enables continuous tracking of physiological parameters, the second allows a visual read-out with the main benefit of no required power supply or processing device.

Furthermore, there exist different approaches with regard to the type of carrier platform used in skin-based sensors, for example, wrist bands, band-aids, patches, temporary tattoos, and the like.

WO03105694 A1 describes a temporary tattoo for testing the sensitivity of skin to chemicals present in cosmetics. The areas with the analytes are in contact with the skin and are protected from getting wiped-off by the water-insoluble layer of the temporary tattoo.

US 6267724 B1 discloses a method of using an implantable diagnostic sensor, the method comprising colorimetric analysis of sub-skin physiological conditions such as pH in subcutaneous fluid.

US 2020363322 A1 describes a wearable pollutant sensor configured to be placed on the skin, wherein the sensor comprises a colorimetric indicator layer capable of sensing airborne particulate pollution in the environment.

US 2009325221 A1 discloses a skin-transferable temporary tattoo decals comprising a colorimetric layer for indicating the presence of targeted analytes, e.g. liquids or gases, in the environment.

WO 2019165244 A1 discloses a temporary tattoo decal comprising a photochromic ink layer that includes a photochromic material that changes color due to UV irradiation.

US 2002074003 A1 discloses a skin-injected tattoo with a photochromic dye that changes color due to UV irradiation.

WO 2018232387 A1 discloses a wearable UV indicator comprising a qualitative indicator of intensity of and exposure to solar ultraviolet radiation.

Despite the advantages of such tools, there exists only few realizations of market-available sweat sensors based on optical techniques so far. This is owed to several challenges when analyzing sweat, such as meager sweat volume, distinct detection of different analytes or personal sweat composite deviations:
**Sweat volume:** Patients, especially in sedentary state, can have sweat rates of subnanoliter per minute per sweat gland. Collection and transport of such little amounts require long-lasting measurements and complicated microfluidic systems with specialized measure, to minimize evaporation of sweat.

**Interference with pH or metabolism:** Typical sensor readings are influenced by the pH level of sweat which changes due to external contaminants or skin excreted components. Further, the analytes concentration within sweat is influenced by the metabolism of sweat glands.

**Individual deviations:** Sweat rate, skin topography, or sweat analyte concentrations vary from one person to another due to physical maturation, hydration, acclimatization, time of the day, age, or region of the body. The complicated correlation of these parameters towards the sweat biomarkers and further to the blood composition needs to be employed to establish a measurement protocol.

As in situ health-care measurements have become of major interest in healthcare diagnostics, there remains a constant need for an inexpensive skin-wearable sweat sensor device which is ultra-thin, ultra-conformal to skin, and enables fast detection of physiological properties of sweat.

It is an object of the invention to provide novel and improved sweat sensors based on optical techniques, which sensors meet the above-mentioned needs and solve the above-mentioned technical problems and challenges, especially the problem of detecting physiological analytes in meager sweat volumes. It is another object of the invention to provide methods for manufacturing a sweat sensor according to the invention and to provide methods of monitoring the health status of an individual by using the sweat sensor according to the invention.

### Description of the Invention

To achieve said objects of the invention, a temporary tattoo sensor for application to an individual's skin and for detecting physiological analytes within sweat of the individual is provided, which may overcome one or more of the foregoing problems associated with known wearable sweat sensors. Furthermore, to achieve said objects of the invention, also methods for manufacturing a temporary tattoo sensor according to the invention and to methods of monitoring the health status of an individual by using the sweat sensor according to the invention are provided.

In one aspect, the present invention provides a temporary tattoo sensor for application to an individual's skin and for detecting physiological analytes within sweat of said individual, wherein the temporary tattoo sensor comprises:
a substrate layer configured to be placed on the skin of the individual and having a first side facing away from the skin and a second side facing the skin, when the temporary tattoo sensor is placed on the skin of the individual;
a colorimetric layer disposed on the second side of the substrate layer, wherein, when the temporary tattoo sensor is placed on the skin of the individual, the colorimetric layer is adjacent to and in direct contact with the skin;
wherein the colorimetric layer comprises a hydrogel matrix and at least one indicator dye;
wherein the hydrogel matrix is configured to accumulate sweat that segregates from the individual's skin and to act as a carrier matrix for immobilizing the at least one indicator dye; and
wherein the at least one indicator dye is configured to visibly change color upon contact with at least one physiological analyte present in the individual's sweat accumulated in the hydrogel.

The invention provides for an inexpensive wearable sweat sensor which is thin and conformal to skin compared to sweat sensors based on wrist bands, patches or band-aids, and there is a minimal dead volume between the skin and the sensor. This enables a comfortable feeling to users wearing the sensor, e.g. for in situ health care monitoring.

The collection and transport of the sweat is achieved through a thin colorimetric layer, preferably having a thickness of 0.1 µm to 10 µm, comprising a hydrogel matrix and indicator dye molecules immobilized by the hydrogel matrix. The sweat is accumulated by the hydrogel matrix and transported to the indicator dye molecules which will adapt due to analytes in the accumulated sweat with a visible color change. The volume between the sweat gland and the colorimetric layer is marginal. Even applying the sensor of the invention on persons with little sweat rates, such as sedated patients, would lead to reliable results within a short period of time. Uniting sweat collection, transport and colorimetric analysis in one ultrathin layer, i.e. the colorimetric layer, and applying the same to the skin via a temporary tattoo-based substrate layer provides the necessary properties to deal with minimal sweat rates.

Previously known tattoo-based sensors are based on an electroanalytical detection and are operated by an electronical read-out strategy [e.g. Windmiller et al, Chem. Commun. 2012, 48, 6794; Guinovart et al, Analyst, 2013, 138, 7031; Bandodkar et al, Analyst 2013, 138, 123], needing supplementary parts, such as power units or electronic read-out devices, which are bulky and, due to required electrical connection, uneasy to handle. In contrast to the known sensors, the temporary tattoo sensor of the invention being based on a tattoo-based colorimetric approach advantageously responses with a color change to physiological properties of sweat, thereby permitting a fast visual read-out and feedback of the state of health without requiring adjunctive technology such as a powering unit or read-out devices. Moreover, with in this invention no electroanalytical but, for the first time, an optical detection strategy combined with temporary tattoos is disclosed, which will not create false output due to deformations.

The invention makes it possible to detect a deterioration in a person's state of health at an early stage in a cost-effective and uncomplicated manner and to take appropriate health-related measures and/or to analyze the state of health in more detail at an early stage of diseases. It will be clear to the skilled person that the information obtained with the temporary tattoo sensors according to the invention may be qualitative or semiqualitative, i.e., the visible color change of the indicator dye is indicative of a deviation from a particular health condition; further analysis and testing is required for a profound quantitative analysis or diagnosis, like measurement of the RGB color values via a software-based application, e.g. via a smartphone-assisted color recognition app, or by more exactly determining the concentration of analytes present in the sweat in a laboratory environment, or by any other diagnostic method that allows a profound diagnosis of a suspected health condition preliminarily detected by means of the colorimetric tattoo sensor of the present invention.

Consequently, the combined features of a temporary tattoo sensor of the present invention solve the important problem of detecting physiological analytes in meager sweat volumes and satisfy the critical need for an inexpensive skin-wearable sweat sensor device which is ultra-thin, ultra-conformal to skin, and enables fast detection of physiological properties of sweat without major technical expense or effort. The invention is not only beneficial in medical or clinical settings, e.g. for monitoring the health status of patients having diabetes or kidney problems, but is also useful in non-medical/non-clinical settings such us home environment, schools, sports facilities like gyms, wellness facilities et cetera, for monitoring personal health, personal wellness, electrolyte loss of athletes, et cetera.

The expression "visible color change" as used herein refers to a color change by aid of an indicator dye that is perceptible by the human eye. The visible color change may be based on all colorimetric methods known to those skilled in the art, including non-enzymatic and enzymatic methods. The term "indicator dye" is to be interpreted broadly and covers all kinds of color reagents known to those skilled in the art, that may be safely applied to the skin of an individual and, in the normal case (e.g. no allergic reaction), does not cause any harm or adverse effects upon contact with the skin. In particular, the broadly to be interpreted term "indicator dye" may include both organic and inorganic color reagents as well as enzymes catalyzing a color reaction, which result in a visible color change in the presence of a physiological analyte present in the individual's sweat. The color intensity depends on the amount of indicator dye molecules within the colorimetric layer. By either increasing the dye density or the layer thickness an appropriate color intensity can be reached. The skilled person is, due to his/her general knowledge and in view of the present disclosure, well able to choose appropriate indicator dyes and their density in the colorimetric layer by way of routine testing.

Basically, known color-changing indicator dyes commonly used in printed diagnostic tools can also suitably be implemented in the present invention; a well-known representative example relates to commercially available urine test strips applied in routine urine examinations to detect urinary tract infections, proteinuria, glucosuria etc. Many of the indicator dyes used in the indicator fields of urine test strips for detecting urine-based analytes are also able to detect and change color upon presence of sweat-related physiological analytes (e.g. pH, glucose, etc.) and, thus, the skilled person is well able to apply these indicator dyes commonly used for testing urine to the present sweat-related invention.

The term "individual" refers to human or animal subjects, preferably human subjects.

The substrate layer is configured to be placed on the skin of the individual and, in order to meet the invention's purpose, has to be very thin and conformal to skin, e.g. flexible or preformed. The skilled person will understand that, due to the orientation of the substrate layer and colorimetric layer towards the skin, the substrate layer must be configured to enable direct visual read-out of a color change, and expediently is a transparent layer. Moreover, the substrate layer preferably is water-repellent or waterproof, breathable (providing the possibility for old sweat to evaporate) and adheres well to an individual's skin. Furthermore, the substrate layer must be configured to allow deposition of the colorimetric layer and, thus, shall show sufficient stability for the intended use. It can be provided that the substrate layer consists of flexible transparent synthetic material. In preferred embodiments, the substrate layer acting as a carrier layer for the colorimetric layer is a temporary tattoo layer, a decal or a medical adhesive layer (e.g. Fixomull^{®} transparent).

Most preferably, the substrate layer is a temporary tattoo layer. Temporary tattoo layers are ultra-thin and transparent layers and, in the normal case, are part of tattoo papers. Tattoo papers are well known, can be customized by depositing an image e.g. by printing and are commercially available from many different manufacturers (e.g. TheMagicTouch Ltd, UK; Sillhouette; Mondo Decal; Papilio; Crafty). Tattoo papers are typically three-layered papers consisting of a tattoo layer (i.e. the substrate layer of the present invention) that is finally released onto the skin, a sacrificial water-dissolvable release layer applied to the temporary tattoo layer and a removable support layer applied to the water-dissolvable release layer.

Thus, in a particularly preferred embodiment of the present invention, the temporary tattoo sensor further comprises a water-dissolvable release layer applied to the first side of the substrate layer; and a removable support paper applied to the water-dissolvable release layer. In this particularly preferred embodiment, the combination of substrate layer, water-dissolvable release layer and removable support paper forms a three-layered water slide decal paper, preferably a tattoo paper, which acts as a deposition substrate for the deposition of the colorimetric layer. For transferring the substrate layer and colorimetric layer onto the skin, the temporary tattoo sensor of this embodiment is placed onto the skin and the removable support paper is removed by dissolving the sacrificial water-dissolvable release layer in water, while the substrate layer and colorimetric layer adhere to the skin and the colorimetric layer is enclosed between the skin and the second side of the substrate layer. Due to their thinness (typically < 1µm thickness) temporary tattoo layers have the great benefit of smoothly following complex topography features including the topography of skin. Therefore, the colorimetric layer deposited on the second side of the substrate layer is in intimate contact with the skin. Preferably, the temporary tattoo layer is breathable, i.e. provides water vapor transport properties to let the skin "breath", providing the possibility for old sweat to evaporate.

The colorimetric layer disposed on the second side of the substrate layer comprises or consists of a hydrogel matrix and at least one indicator dye. The hydrogel matrix is configured to accumulate sweat that segregates from the individual's skin and to act as a carrier matrix for immobilizing the at least one indicator dye. Hydrogels are well known in the art and are polymer matrixes of hydrophilic molecule chains, connected with cross-linkers. Increasing the cross-linker density stabilizes the hydrogel and in trade off the swelling capacity is reduced.

As will be explained in more detail further below, the molecules of the at least one indicator dye are immobilized in that they are either embedded within the hydrogel matrix or are covered by a layer of the hydrogel matrix. As will be also explained further below, the colorimetric layer may be deposited by either a simultaneous or sequential deposition of the indicator dye molecules and the hydrogel, wherein solvent-based deposition techniques, e.g. spin-coating and solutions polymerization techniques, vacuum-based deposition techniques, or combinations thereof may be applied.

In certain embodiments it can be provided that the at least one indicator dye changes from a first visible color to a second visible color, or vice versa, upon contact with the at least one physiological analyte present in the individual's sweat accumulated in the hydrogel.

In certain embodiments it can be provided that the at least one indicator dye changes from transparent to a visible color, or vice versa, upon contact with the least one physiological analyte present in the individual's sweat accumulated in the hydrogel.

There is a broad variety of indicator dyes with a selective color change to certain physiological analytes present in sweat, which indicator dyes are known to those skilled in the art and can be suitably applied in the present invention. As mentioned above, the broadly to interpreted term "indicator dye" may include both organic and inorganic color reagents as well as enzymes catalyzing a color reaction, which result in a visible color change in the presence of a physiological analyte present in the individual's sweat. As also mentioned above, known color-changing indicator dyes commonly used in printed diagnostic tools can also suitably be implemented in the present invention, for example the color-changing indicator dyes used in commercially available urine test strips applied in routine urine examinations. Preferably, the at least one indicator dye that is configured to visibly change color upon contact with the at least one physiological analyte is selected from the group consisting of: pH, calcium, chloride, corticosteroids, creatinine, urea, amino acids, glucose, lactate, and combinations thereof.

The following Table 2 shows representative examples of different color changing responsive indicator dyes (non-enzymatic; enzymatic) together with the corresponding physiological analyte in sweat. The skilled person will understand that the responsive indicator dyes given in Table 2 are merely exemplary and shall not be construed restrictive.

**Table 2: responsive indicator dyes and respective physiological analytes in sweat**

| **Responsive indicator dye** | **Physiological analyte in sweat** |
|---|---|
| bromocresol purple, bromocresol green, litmus, methyl red | pH |
| Examplary colorimetric methods are described by Zhang et al, Lab Chip, 2019, 19, 1545 and by Choi et al, ACS Sens., 2019, 4, 379-388 | |
| o-cresolphthaleine, phthalein purple | calcium |
| silver chloranilate [e.g. methods described by Choi et al, ACS Sens., 2019, 4, 379-388] | chloride |
| enzymatic colorimetric assays using creatininase/creatinase; [e.g. methods described by Zhang et al, Lab Chip, 2019, 19, 1545] | creatinine |
| picrate (Jaffe method) | |
| enzymatic colorimetric assays using urease [methods described e.g. by Zhang et al, Lab Chip, 2019, 19, 1545] | urea |
| enzymatic colorimetric assays using glucose oxidase [e.g. methods described by Choi et al, ACS Sens., 2019, 4, 379-388] | glucose |
| enzymatic colorimetric assays using lactate oxidase [e.g. methods described by Choi et al, ACS Sens., 2019, 4, 379-388] | lactate |
| CHOD-PAP method (cholesterol oxidase, phenol + aminophenazone/4-chlorophenol) | corticosteroids |
| sulfuric acid, Porter-Silber reagent, Prussian blue, and blue tetrazolium [see also Tu et al, ACS Omega, 2020, 5, 8211-8218] | |
| Colorimetric methods for detecting corticosteroids are described in Sefid-Sefidehkhan et al, Chemical Papers, 2022, 76, 4627 | |
| "ninhydrin-test" [see e.g. method described by Guembe-Garcia et al, Sensors and Actuators, B:Chemical, 2021, 335, 129688] | amino acids |

In further preferred embodiments, it can be provided that the temporary tattoo sensor according to the invention further comprises at least one additional indicator dye comprised in the colorimetric layer and immobilized by the hydrogel matrix, wherein the at least one additional indicator dye is configured to visibly change color upon detecting at least one further skin- or sweat-related parameter, and wherein the at least one additional indicator dye is selected from the group consisting of: a thermochromic indicator dye configured to visibly change color depending on the skin's temperature, a hydrochromic indicator dye configured to visibly change color depending on the skin's humidity or sweat rate, a photochromic indicator dye configured to visibly change color depending on the UV exposure, and combinations thereof. Thermochromic indicator dyes, hydrochromic indicator dyes and photochromic indicator dyes that may be suitably applied in the present invention are commercially available and known to those skilled in the art.

Representative, non-restrictive examples of thermochromic indicator dyes suitable for being applied in the present invention are a ternary cholesteric liquid crystalline mixture of cholesteryl oleyl carbonate, cholesteryl nonanoate, and cholesteryl 2, 4-dichlorobenzoate. For example, a thermochromic indicator dye for colorimetric analysis of temperature of sweat has been described by Choi et al, ACS Sens., 2019, 4, 379-388.

Representative, non-restrictive examples of hydrochromic indicator dyes suitable for being applied in the present invention are oxazolidines, oxazines, and photosensitive Prussian blue. For example, a hydrochromic indicator dye for colorimetric analysis has been described by Bilgin and Backhaus, J. Print Media Technol. Res., 2020, 3, 137-143; Bilgin and Backhaus, Advances in Printing and Media Technology, Vol. XLIV (IV) - 3B: Printing for Packaging, 89-96.

Representative, non-restrictive examples of photochromic indicator dyes being suitably applied in the present invention are diarylethene, spiropyran, spirooxazine, naphthopyran. For example, photochromic indicator dyes for colorimetric analysis of UV exposure have been described by Pardo et al, Chem. Soc. Rev., 2011, 40, 672.

The at least one additional indicator dyes, selected from the group consisting of thermochromic indicator dyes, hydrochromic indicator dyes and photochromic indicator dyes, and combinations thereof, can be useful for calibration purposes. For example, for calibration purposes, the at least one additional indicator dye which is selected from thermochromic indicator dyes, hydrochromic indicator dyes, photochromic indicator dyes and combinations thereof may be deposited directly aside the indicator dye spot and a white colored spot. If the sensor is analyzed by a camera or a smart phone the white spot is used as a reference. Only the change of RGB values from the white spot to the indicator dyes or thermochromic indicator dyes or hydrochromic indicator dyes or photochromic indicator dyes will be taken as the color of the sensor. It can be expected that some of the mentioned indicator dyes do net selectively respond to only one target analyte but instead react with a cross-sensitivity to different analytes or environmental condition (e.g. temperature, humidity). In such cases it is of need to apply several temporary tattoo sensors of several different indicator dyes and or thermochromic indicator dyes and or hydrochromic indicator dyes and or photochromic indicator dyes. By analyzing them simultaneously and knowing the cross-sensitivity of each sensor towards the analytes and or the environmental conditions the analyte concentration can be calculated.

In specific embodiments, it can be provided that each of the at least one indicator dye and, if present, each of the at least one additional indicator dye, is separately and visually distinguishably disposed in at least one discrete area of the colorimetric layer, wherein preferably the colorimetric layer comprises a colorimetric dye array comprising dye spots of said at least one indicator dye and at least one additional indicator dye, respectively, immobilized by the hydrogel matrix. In these specific embodiments, the colorimetric layer may be designed as a color responsive sensor array, wherein dye spots of different indicator dyes and, if present, different additional indicator dyes are disposed in the hydrogel matrix, for detecting different physiological analytes and further skin- or sweat-related parameters at once. The sensor array may be either read-out visually or software-assisted, e.g. RGB color values may be tracked via a software-based application, e.g. via a smartphone-assisted color recognition app. Color responsive sensor arrays for detecting of different analytes in sweat, which may be suitably implemented in the present invention, have been reported earlier, e.g. by the group of Zhang et al, 2021, Journal of Materials Chemistry C, 9(41), 14938-14945, describing an adhesive hydrogel patch for colorimetric sweat detection, the patch comprising an array of dye spots detecting different analytes such as pH, glucose, chloride, and calcium in human sweat.

Preferably, the hydrogel is selected from the group consisting of: poly-hydroxyethylacrylate (pHEMA), poly N-vinylcaprolactam /pNVCL), poly methacrylic acid (pMAA) pure and in combination with crosslinkers, such as ethylenglycoldimethacrylate (EGDMA), ethylenglycoldiacrylate (EGDA), diethylglycoldivinylether (DEGDVE), tetravinyltetramethyloctaciclosiloxane (V4D4), trivinyltrimethylesaciclosiloxane (V3D3).

In certain embodiments the molecules of the at least one indicator dye and, if present, the molecules of the at least one additional indicator dye are either embedded within the hydrogel matrix. These embodiments may be obtained by solvent-based deposition techniques, like spin-coating and a solution polymerization technique, or by vacuum-based deposition techniques, e.g. simultaneous vacuum deposition of evaporated indicator dyes (via physical vapor deposition, PVD) with in-situ synthesized hydrogel (via initiated chemical vapor deposition, iCVD).

In other embodiments the molecules of the at least one indicator dye and, if present, the molecules of the at least one additional indicator dye are covered by a layer of the hydrogel matrix. These embodiments may be obtained by solvent-based deposition techniques or by vacuum-based deposition techniques, alone or in combination with each other. For example, the indicator dye is deposited first, e.g. by printing, spin coating or physical vapor deposition (PVD), followed by depositing the hydrogel via a solution polymerization technique or by initiated chemical vapor deposition (iCVD).

Preferably, the colorimetric layer has a thickness of 0.1 µm to 10 µm.

In the following, methods for manufacturing temporary tattoo sensors according to the invention are described. The colorimetric layer may be deposited on the second side of the substrate layer by either a simultaneous or sequential deposition of the molecules of the at least one indicator dye and the hydrogel matrix. Because of the delicate nature a tattoo layer (or tattoo paper), if used as a substrate, distinct delivery methods for depositing the colorimetric layer are required. In the present invention, solvent-based deposition techniques, vacuum-based deposition techniques, or combinations thereof are useful for depositing the colorimetric layer on the second side of the substrate layer, which preferably is a tattoo layer. As a representative example, poly-hydroxyethyl-methacrylate (pHEMA) may be utilized in the present invention as a suitable hydrogel matrix. pHEMA can be deposited onto the substrate layer via solution-based methods or vacuum-based methods. Furthermore, it is known to use pHEMA for immobilizing dyes for colorimetric body sensors [see e.g. Choi et al, ACS Sensors, 2019, 4, 379-388]. In addition, in a previous study of the applicant, it was shown that copolymers of pHEMA and ethylene glycol dimethacrylat (EGDMA) with tunable mesh sizes (the mesh size being the length within a polymer matrix from one to another cross-link connection) can be delivered via iCVD [Unger et al, Macromol. Chem. Phys., 2016, 217, 2372]; thus, such copolymers can be advantageously used to immobilize the indicator dye molecules described herein within a hydrogel matrix of mesh sizes smaller than the size of the respective indicator dye molecules.

In a further aspect, the present invention relates to a method for manufacturing a temporary tattoo sensor as defined and described in the present disclosure, the method comprising depositing the colorimetric layer on the second side of the substrate layer by using solvent-based deposition techniques and/or vacuum-based deposition techniques.

As a solvent-based deposition routine, spin-coating with appropriate solvents, as well as screen printing or inkjet printing, and solution polymerization techniques may be applied, thereby casting the indicator dye molecules and hydrogel matrix onto the second side of the substrate layer. Especially enzyme-based indicator dyes, which are large molecules, may unlikely go into vapor phase without degrading, and, hence, for such dyes, solvent-based deposition techniques are preferred.

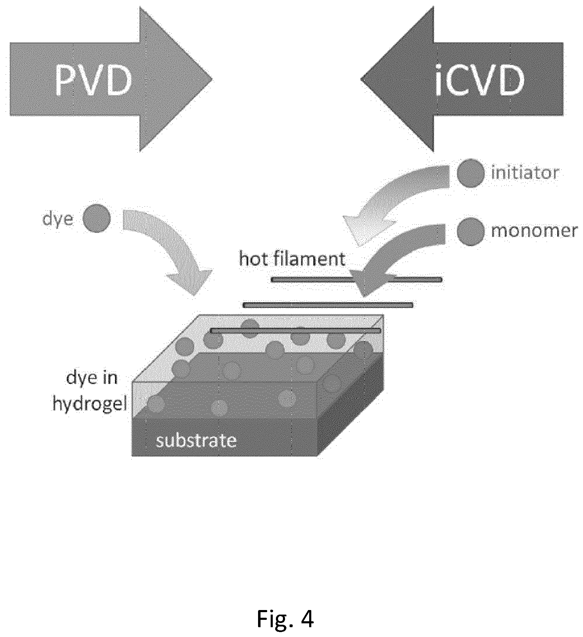

Vacuum-based deposition techniques include physical vapor deposition (PVD) and initiated chemical vapour deposition (iCVD). The main advantage of vacuum-based deposition techniques is the absence of toxic or material degrading solvents. Further, the utilized techniques require no ultra-high vacuum, no elevated substrate temperature and no plasma. This is taken advantage of in terms of delivering the colorimetric layer onto delicate substrates such as tattoo paper, which would otherwise degenerate at harsh temperature conditions. Within the scope of the present invention, a novel approach of simultaneous vacuum deposition of evaporated indicator dyes (via physical vapor deposition, PVD) with in-situ synthesized hydrogel (via initiated chemical vapour deposition, iCVD) is disclosed and made available to the public (see also example 2 as well as Fig. 4 and pertaining description below).

In certain embodiments the method comprises: a step (i) of applying the at least one indicator dye dissolved in a solvent and, if present, the at least one additional indicator dye dissolved in a solvent, to the second side of the substrate layer via a solvent-based deposition technique, to form an indicator dye layer; or of applying the at least one indicator dye and, if present, the at least one additional indicator dye to the second side of the substrate layer by a vacuum-based deposition technique, to form an indicator dye layer; wherein step (i) is followed by a step (ii) of creating a layer of the hydrogel matrix on the second side of the substrate layer and on top of the deposited indicator dye layer, by means of a solution polymerization technique (i.e. a solvent-based deposition technique) or by means of a vacuum-based deposition technique. Accordingly, by depositing the indicator dye layer and the hydrogel layer independently from each other either by solvent-based deposition techniques or by vacuum-based deposition techniques, the colorimetric layer is formed.

In certain embodiments, the method comprises the steps of: applying the at least one indicator dye dissolved in a solvent and, if present, the at least one additional indicator dye dissolved in a solvent, to the second side of the substrate layer, to form an indicator dye layer, followed by creating a layer of the hydrogel matrix on the second side of the substrate layer and on top of the deposited indicator dye layer, by means of a solution polymerization technique.

In certain embodiments, the method comprises depositing the colorimetric layer on the second side of the substrate layer by using vacuum-based deposition techniques, including physical vapor deposition (PVD) and initiated chemical vapor deposition (iCVD).

Conformal deposition of the colorimetric layer, especially the hydrogel, is preferred in order to ensure minimal dead volume between the sweat gland and the colorimetric layer, when the temporary tattoo sensor is placed on the individual's skin. Conformal deposition ensures that the sweat is effectively accumulated by the hydrogel matrix and transported to the indicator dye molecules which will then adapt due to analytes in the accumulated sweat with a visible color change. Conformal deposition of the hydrogel matrix can be carried out by initiated chemical vapor deposition (iCVD). This technique can allow deposition of high quality hydrogels directly from a vapor phase. Controlling the pm/psat (monomer pressure at the substrate temperature versus its saturation pressure) of the inflowing monomers gives a hand on the cross-linker density within the hydrogel, which further defines the mesh size. The mesh size is a parameter that defines average spacing between cross-linkers within the hydrogel influencing the diffusion properties of the hydrogel. While dye molecules need to be encapsulated (no allowed diffusion) sweat analytes should still be able to pass the meshes of the hydrogel.

In a particularly preferred embodiment, the colorimetric layer is created on the second side of the substrate layer by co-depositing (i) the at least one indicator dye and, if present, the at least one additional indicator dye, by means of Physical Vapor Deposition (PVD) and (ii) the hydrogel matrix by means of initiated Chemical Vapor Deposition (iCVD) on the second side of the substrate layer. As mentioned above, this is a novel approach of simultaneous vacuum deposition in one vacuum chamber of evaporated indicator dyes (via physical vapor deposition, PVD) with in-situ synthesized hydrogel (via initiated chemical vapor deposition, iCVD) on delicate or fragile substrate layers, especially tattoo layers, wherein this approach, as per the inventors' and applicant's best knowledge has not yet be made available to the public before (see also example 2 as well as Fig. 4 and pertaining description below).

In a further aspect, the present invention relates to a method of monitoring the health status of an individual by measuring analytes within the individual's sweat, comprising the consecutive steps of:
- providing a temporary tattoo sensor as claimed and described in this disclosure;
- applying the temporary tattoo sensor on the individual's skin, wherein the colorimetric layer disposed on the second side of the substrate layer is brought in direct contact with the skin, and
- monitoring the temporary tattoo sensor if a visible color change has occurred.

Thus, this method of monitoring the health status makes it possible to detect a deterioration in a person's state of health at an early stage in a cost-effective and uncomplicated manner and to take appropriate health-related measures and/or to analyze the state of health in more detail at an early stage of diseases.

In one embodiment, said method of monitoring the health status of an individual by measuring analytes within the individual's sweat by using a temporary tattoo sensor according to the invention comprises monitoring the status of a skin wound of an individual, wherein the temporary tattoo sensor is placed onto the skin wound, wherein the method comprises monitoring the temporary tattoo sensor if a visible color change with respect to at least one of the following physiological analytes and skin- or sweat-related parameters, respectively has occurred: pH, urea, glucose, amino acids, calcium, chloride, corticosteroids, creatinine, lactate, temperature, and humidity of the skin wound.

The aspect of tracking the status of wounds via color-changing indicator dyes is well established and a well-known research topic. The review paper by Sun et al, Biosensors, 2022, vol. 1, issue 12, presents an introduction to wearable sensors for wound monitoring. It explains the physiological key markers of chronic wounds. Though the microenvironment of wounds are very complicated, certain markers are also accessible with a colorimetric approach. Especially via colorimetric indicator dyes swabs, cotton cloth, membranes or hydrogels for wounds were already functionalized to track pH, glucose amino acids or temperature of wounds. The skilled person is, due to his/her general knowledge and in view of the present disclosure, well able to choose appropriate indicator dyes for a temporary tattoo sensor according to the invention being functionalized with a hydrogel immobilizing the indicator dye molecules, in order to perform the method of monitoring the status of a skin wound. By the method of monitoring the status of a skin wound of an individual by using a temporary tattoo sensor according to the invention, users may be able to track the following:
- A sudden rise of the pH level: typically caused by bacterial infection.
- Glucose: for wounds of diabetic patients
- Amino acids: for indirectly measuring the enzymatic activity
- An increase of temperature: often reflects a bacterial infection
- A decrease of temperature: may be related to ischemia.
- The humidity of the wound: if the humidity is too little, positive biochemical reactions are limited because they need liquid media, while on the other hand too high levels of wound exudate may lead to wound expansion.

In the following, to further demonstrate the present invention, illustrative and non-restrictive embodiments and examples including experimental data are given and discussed.

### Brief Description of the Drawings

Fig. 1 shows Table 1 illustrating the connection of biomarkers of sweat with health conditions (adapted from reference Moonen et al, View, 2020, 1, 20200077)
Fig. 2 shows a schematic illustration of an embodiment of a temporary tattoo sensor according to the invention;
Fig. 3 shows a schematic illustration of different embodiments of the colorimetric layer of the temporary tattoo sensor of Fig. 2, wherein the molecules of the least one indicator dye are immobilized by the hydrogel matrix by either (a) embedding the molecules of the least one indicator dye within the hydrogel matrix or (b) covering the molecules of the least one indicator dye by a layer of the hydrogel matrix;
Fig. 4 shows a schematic illustration of the co-deposition of evaporated indicator dye molecules and an initiated chemical vapor deposited hydrogel polymer on a substrate layer;
Fig. 5 shows the results of a test series, where indicator dyes responsive to pH, temperature and UV were dissolved in ethanol and then spin-coated on tattoo paper;
Fig. 6 shows the pH-colorimetry results of a colorimetric layer comprising a hydrogel matrix with immobilized methyl red pH indicator dye;
Fig. 7A shows optical microscopy images of a silicon sample (not of the invention) comprising a colorimetric layer comprising a hydrogel matrix with immobilized methyl red pH indicator dye;
Fig. 7B shows optical microscopy images of a glass sample (not of the invention) comprising a colorimetric layer comprising a hydrogel matrix with immobilized methyl red pH indicator dye;
Fig. 7C shows optical microscopy images of a tattoo paper sample (according to the invention) comprising a colorimetric layer comprising a hydrogel matrix with immobilized methyl red pH indicator dye, wherein the shown area of the sample was not treated with a pH-solution;
Fig. 7D shows optical microscopy images of a silicon sample (not of the invention) comprising a colorimetric layer comprising a hydrogel matrix with immobilized methyl red pH indicator dye, wherein the shown area of the sample was treated with a pH-solution;
Fig. 8 shows the results of a transmission photospectrometry measurement of a glass sample (not of the invention) comprising a colorimetric layer comprising a hydrogel matrix with immobilized methyl red pH indicator dye;
Fig. 9 shows the laser interferometry measurement during deposition of a colorimetric layer on a substrate by a combined PVD-iCVD process according to example 2.

### Detailed Description of Embodiments and Examples

In the following figures identical reference signs refer to identical features unless expressly depicted otherwise. The reference signs are only for informational purpose and do not delimit the scope of protection.

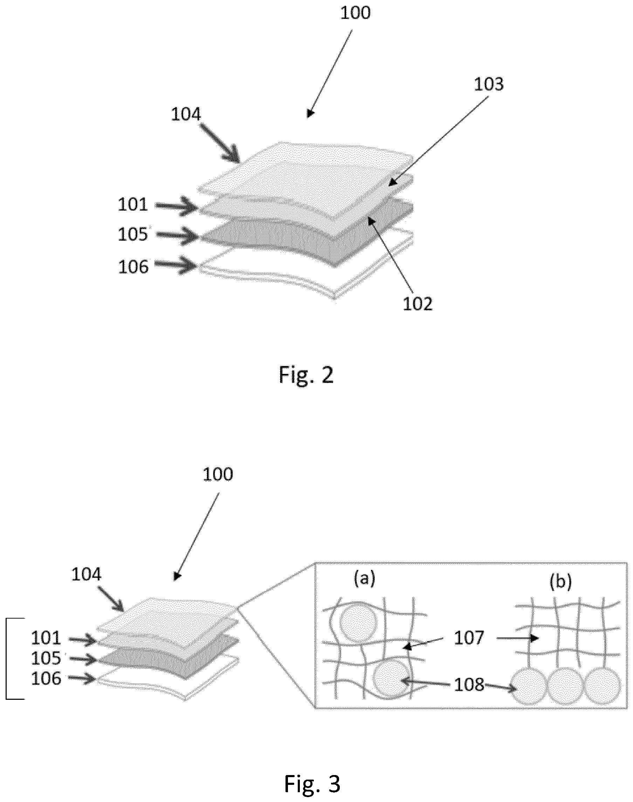

Fig. 2 shows a schematic illustration of an embodiment of a temporary tattoo sensor 100 according to the invention. The temporary tattoo sensor 100 comprises a substrate layer **101** configured to be placed on the skin of the individual and having a first side **102** facing away from the skin and a second side **103** facing the skin, when the temporary tattoo sensor 100 is configured to be placed on the skin of the individual. The temporary tattoo sensor 100 further comprises a colorimetric layer **104** disposed on the second side 103 of the substrate layer 101, wherein, when the temporary tattoo sensor 100 is placed on the skin of the individual, the colorimetric layer 104 is adjacent to and in direct contact with the skin. The colorimetric layer 104 comprises a hydrogel matrix and at least one indicator dye. The hydrogel matrix is configured to accumulate sweat that segregates from the individual's skin and to act as a carrier matrix for immobilizing the at least one indicator dye. The at least one indicator dye is configured to visibly change color upon contact with at least one physiological analyte present in the individual's sweat accumulated in the hydrogel. The temporary tattoo sensor 100 further comprises a water-dissolvable release layer **105** applied to the first side 102 of the substrate layer 101 and a removable support paper **106** applied to the water-dissolvable release layer 105. The combination of substrate layer 101, water-dissolvable release layer 105 and removable support paper 106 forms a three-layered tattoo paper, which acts as a deposition substrate for the deposition of the colorimetric layer 104. For transferring the substrate layer 101 and colorimetric layer 104 onto the skin, the temporary tattoo sensor 100 shown in Fig. 2 is placed onto the skin and the removable support paper 106 is removed by dissolving the sacrificial water-dissolvable release layer 105 in water, while the substrate layer 101 and colorimetric layer 104 adhere to the skin and the colorimetric layer 104 is enclosed between the skin and the second side 103 of the substrate layer 101.

Fig. 3 shows a schematic illustration of different embodiments of the colorimetric layer of the temporary tattoo sensor of Fig. 2. The colorimetric layer 104 comprises a hydrogel matrix **107** and at least one indicator dye **108.** The molecules of the least one indicator dye 108 are immobilized by the hydrogel matrix 107 by either (a) embedding the molecules of the least one indicator dye 108 within the hydrogel matrix 107 or (b) covering the molecules of the least one indicator dye 108 by a layer of the hydrogel matrix 107.

Fig. 4 shows a schematic illustration of the co-deposition of evaporated dye molecules and initiated chemical vapor deposited hydrogel polymer on a substrate layer. Fig. 4 illustrates, how the colorimetric layer is created on the second side of the substrate layer (indicated as "substrate") by co-depositing (i) the at least one indicator dye and, if present, the at least one additional indicator dye, by means of Physical Vapor Deposition (PVD) and (ii) the hydrogel matrix by means of initiated Chemical Vapor Deposition (iCVD) on the second side of the substrate layer. Within the PVD process, the dye molecules are heated in a suitable cell, e.g. a powered tungsten boat, until evaporation can be observed, e.g. measured by a quartz micro balance. The dye molecules will then adsorb on the substrate and condense. Within iCVD (for forming the hydrogel matrix), the initiator and monomer units enter the chamber as vapors. At a hot filament (approx. 150°C - 200°C), the initiator decomposes and forms radicals. The momomer units (as well as the dye molecules) are not affected by the hot filament as their decomposition temperature in vacuum is beyond 250°C. The monomer units as well absorbs at the substrate surface, i.e. the second side of the substrate layer. When an initiator radical strives the substrate surface, it reacts together with the monomer in a free radical chain polymerization reaction. As mentioned already, the simultaneous vacuum deposition in one vacuum chamber of evaporated indicator dyes via PVD with in-situ synthesized hydrogel via iCVD on delicate or fragile substrate layers, especially tattoo layers, as per the inventors' and applicant's best knowledge, has not yet be made available to the public before. Hydrogels being used as a matrix for immobilizing the indicator dye molecules in the present invention, are cross-linked polymer networks, which means, that their polymer chains are occasionally connected among each other. In iCVD, this can be for example introduced by depositing a co-polymer consisting of a hydrophilic functional group (e.g. 2-hydroxy ethyl methacrylate) and a cross-linker unit (e.g. di(ethylene glycol)divinyl ether or ethylene glycol dimethacrylate (EGDMA)). Increasing the amount of cross-linker results in a smaller mesh-size, which is beneficial in terms of immobilizing the dye molecules, but in trade off causes less swelling.

### Example 1: Spin-coating of indicator dyes onto tattoo paper

### 1.1 Aim

Within this test series, indicator dyes responsive to pH, temperature and UV were dissolved in ethanol and then spin-coated on tattoo paper.

It is noted that in these test series no hydrogel matrix was deposited and, thus, the temporary tattoo sensors described in this example are not according to the invention. However, these test series show that the delicate tattoo layer of a tattoo paper did not get compromised by spin-coating the indicator dyes onto its surface and is still transferrable. Furthermore, the deposited layers of indicator dyes proved excellent responsiveness when placed on the skin of individuals with normal sweat volumes, indicating no degeneration of the dyes.

### 1.2 Materials, methods, and results

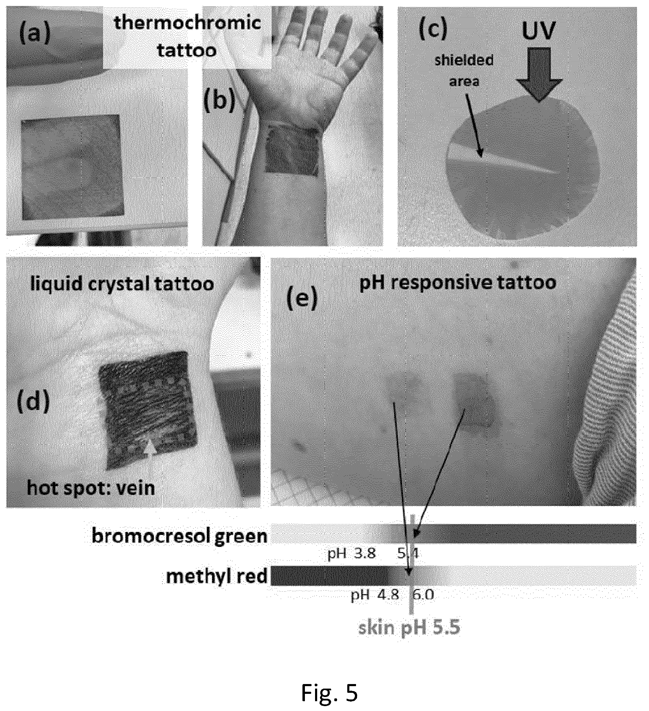

The results of these tests are given in Fig. 5, showing photographs of the tattoo layers with the different spin-coated indicator dyes responsive to pH, temperature and UV, which have been transferred onto human skin.

### 1.2.1 Spin-coated thermochromic indicator dye on tattoo paper

Thermochromic indicator dye (SFXC, UK, www.sfxc.co.uk; Deep Purple to Neon Pink thermochromic pigments) was dissolved in ethanol at a concentration 0.15 g/ml and spin coated while rotating with a speed of 50 rps and a volume of 0.15 ml on the tattoo layer of a MagicTouch tattoo paper (www.themagictouch.de;"Tattoo 2.1" tattoo paper). The functionalized tattoo layer of the tattoo paper was then placed on the skin of an individual (finger, arm).

Fig. 5/image (a) shows the tattoo layer functionalized with spin-coated SFXC thermochromic indicator dye and placed on a finger of an individual. The SFXC thermochromic indicator dye reacted with a color change from magenta to pink due to increase in heat when pressing the finger.

Fig. 5/image (b) shows the tattoo layer functionalized with spin-coated SFXC thermochromic indicator dye and placed on an arm of an individual. The colormap represents the local heat distribution and revealed the veins as hot spots under the skin, thereby demonstrating high spatial resolution.

### 1.2.2 Spin-coated photochromic UV-responsive indicator dye on tattoo paper

Photochromic UV-responsive indicator dye (SFXC, UK, www.sfxc.co.uk; Photochromic Ink) was dissolved 1mg/ml in ethanol and then spin-coated 6 times upon each other with a speed of 50 rps and a volume of 0.14 ml on the tattoo layer of a MagicTouch tattoo paper (www.themagictouch.de;*"*Tattoo 2.1*"* tattoo paper). The functionalized tattoo layer of the tattoo paper was then placed on the skin of an individual.

Fig. 5/image (c) shows the tattoo layer functionalized with spin-coated SFXC photochromic UV-responsive indicator dye and placed on the skin of an individual. When the sample is illuminated with UV, the color changed reversibly from transparent to blue. Shielded areas instead remained transparent.

### 1.2.3 Spin-coated thermochromic indicator dye on tattoo paper

Thermochromic liquid crystal (Anself SA, MAJOR DIJIT Thermo Chromic Liquid (used as delivered)), obtained via
https://www.amazon.de/gp/product/B086PPJ35G/ref=ppx_yo_dt_b_asin_title_o08_s00?ie =UTF8&psc=1) was spin-coated 6 times upon each other with a speed of 50 rps and a volume of 0.25 ml on the tattoo layer of a MagicTouch tattoo paper (www.themagictouch.de;*"*Tattoo 2.1*"* tattoo paper). The functionalized tattoo layer of the tattoo paper was then placed on the skin of an individual.

Fig. 5/image (d) shows the tattoo layer functionalized with the spin-coated thermochromic indicator dye (*"*liquid crystal tattoo*"*) and placed on a black painted (for better contrast) skin of an individual. The visible color change was caused by the hot temperature by inner blood vessels. The colors represented the heat map and the veins were visible.

### 1.2.4 Spin-coated pH-responsive indicator dye on tattoo paper

Methyl red dissolved in ethanol (2.5 mg/ml) was spin-coated with a speed of 90 rps and a volume of 0.07 ml on the tattoo layer of a MagicTouch tattoo paper
(www.themagictouch.de;*"*Tattoo 2.1*"* tattoo paper). Bromcresol greed dissolved in ethanol (7 mg/ml) was spin-coated with a speed of 90 rps and a volume of 0.08 ml on the tattoo layer of a MagicTouch tattoo paper (www.themagictouch.de;"Tattoo 2.1" tattoo paper). Both functionalized tattoo layer samples of the tattoo paper were then placed on the skin of an individual.

Fig. 5/image (e) shows the two tattoo layers functionalized with the respective spin-coated pH-responsive indicator dye (left: methyl red; right: bromocresol green) by spin-coating and placed on the skin of an individual. Comparing the obtained color of the respective pH-responsive indicator dye with the associated pH, resulted in a pH of 5.5 for both pH-responsive indicator dyes, which is the typical pH of skin.

**Example 2:** Simultaneous vacuum deposition of evaporated indicator dyes (via physical vapor deposition, PVD) with in-situ synthesized hydrogel (via initiated chemical vapour deposition, iCVD.

### 2.1 Aim

Chemical vapor deposition ( CVD) is one of the most important processes used in semiconductor fabrication or thin film deposition in general. In this experiment, initiated chemical vapor deposition, iCVD, was used to deposit thin hydrogel polymer films doped with the pH indicator dye methyl red (i.e. a colorimetric layer according to the invention) on a tattoo paper substrate. Then, the thin film thickness was measured with ellipsometry and UV spectroscopy was used to measure the light transmittance. To gather information about surface properties, optical microscopy was applied. At last, it was tried to observe the change of color when exposing the pH-sensitive indicator dye to different acids and bases. To do the doping of the polymer with indicator dye evaporation of the indicator dye via a physical vapor deposition (PVD) process was applied. This evaporation PVD process was carried out simultaneously to the iCVD process to achieve a homogenous doping of the hydrogel polymer with indicator dye molecules.

### 2.2 Materials and methods

The following devices were used to perform the experiment and are given in the following *Table 3:*

| Device | Manufacturer | ' Type/Device Number |
|---|---|---|
| Water cooling system | Thermo-Scientific | ACCEL 500LC |
| Rotary vane vacuum pump | Pfeiffer Vacuum | DUO 65 |
| Laser | ThorLabs | HNLS 00811-EC |
| Power/Current supply | Heinzinger | PTN 350-5 |
| Throttle valve | MKS | - |
| Manometer | MKS | 626C01TDE |
| Preassure controller | MKS | 600 Series |
| Thermocouple | Omega | OM-HL-EH-TC |
| Ellipsometer | J.A.Woollam | ESM300 |
| Heating pad | built by TU Graz | - |
| Reactor | built by TU Graz | - |
| Optical Microscope | OLYMPUS | BX51 |
| UV Spectrometer | SHIMADZU | UV-1800 |

The reactor used for performing iCVD and PVD was home-built by the applicant Technische Universität Graz (TU Graz) and comprised a vacuum chamber for arranging the samples and heating wire-filaments.

The following materials were used to perform the experiment and are given in the following *Table 4:*

| **Item/Chemical** | **Type/Abbrev.** | **Add. Info** |
|---|---|---|
| Silicon wafer shards | | Different forms and sizes |
| Glas substrate | | 26x80 mm |
| Tatoo-Paper | TheMagicTouch | 26x26 mm |
| Tert-Butyl Peroxide | TBPO | Dep. Temp. 20-30 °C, initiator |
| (Hydroxyethyl)methacrylate | HEMA | Dep. Temp. 75 °C, polymere |
| Di (ethylene glycol) divinyl ether | DEGDVE | Dep. Temp. 70 °C, crosslinker |
| Methyred | | Red dye |

Furthermore, bases and acids having pH levels ranging from 4 to 9 were used.

Deposition of thin hydrogel polymer films doped with the pH indicator dye methyl red by using simultaneous iCVD and PVD:
The iCVD and PVD deposition processes for co-deposition of the hydrogel matrix and the pH indicator dye methyl red were simultaneously performed in the reactor. As supports for depositing the colorimetric layer, tattoo paper and, for evaluation of film thickness and composition, also a glass support and two silicon wafers were placed in the vacuum chamber of the reactor. One of the silicon wafers was positioned such that the laser of the reactor's thickness monitoring system is reflected of its surface to the laser detector. Once the substrate samples were in place, a rig with the heating wire-filaments was placed on top and a metal boat comprising the methyl red pH indicator dye was in suspension by the heating wire-filaments. For depositing the hydrogel matrix via iCVD, as precursor a mixture of three materials was used, namely TBPO (initiator), HEMA (monomers), DEGDVE (crosslinker), wherein containers comprising the initiator, the monomers and the crosslinkers were connectable to the vacuum system of the reactor via needle valves. The temperature of the precursor chemicals was set as needed with the reactor controller, which was 75°C for HEMA and 70°C for DEGDVE. The vacuum pump was started and was active throughout the entire process. Next, the precursor flow rates were set by means of needle valves. Flow rates of 0.375±0.004sccm for HEMA and 0.61±0.07sccm for DEGDVE, corresponding to 35% DEGDVE and 65% HEMA respectively, were set. The flow rate of the initiator TBPO was set at 1.02 ± *0.06sccm.*

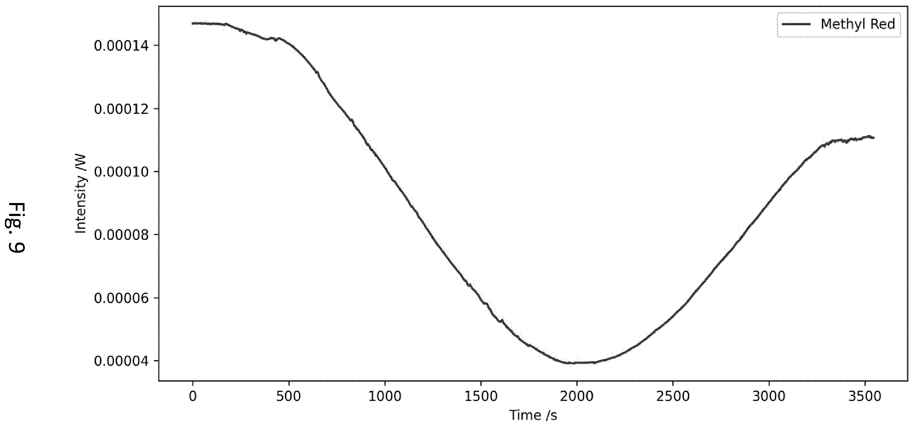

For simultaneous deposition of the hydrogel matrix and the pH indicator dye methyl red as a thin film (colorimetric layer) on the substrate samples, the pressure in the reactor was increased to 200 mTorr. To start the thickness monitoring by laser inferometry the associate program was started. The current supply of the heating wire-filaments was turned on and set to 1.1A. Then, the timer was started and the increase in thickness of the deposited thin layer with the aid of the laser interferometry program was monitored; the laser interferometry measurement is shown in Fig. 9. The targeted thin film layer thickness was 200 nm. After the targeted layer thickness was reached, the deposition process was terminated, wherein the monomer (HEMA) and crosslinker (DEGDVE) valves were shut first 53 minutes after starting the deposition process, followed by the initiator (TBPO) valve three minutes thereafter. Another minute later the reactor's throttle valve was opened and, after the pressure in the reactor chamber decreased to its minimum, the reactor was opened.

The thin films deposited on the substrate samples (tattoo paper, glass substrate, silicon wafers) were subsequently characterized.

### 2.3 Characterization of thin films of hydrogel matrix doped with methyl red molecules

### Ellipsometry

Ellipsometry measurements were performed on the silicon wafer samples and the glass sample. The latter, however, turned out to be just as error prone as it was expected to be. To perform the measurement, the sample was placed at the centre of the ellipsometers probe dish. Upon prompting the measurement within the program associated with the ellipsometer, the user is tasked to adjust the levelling of the probe dish such that equal light intensities hit the four quadrants of the detector. Also, the angels at which measurements were performed are set. In this experiment the sweep started at 65 degrees and ended at 75 degrees with a step size of five degrees. To analyse the measured data and to extract the thickness, a model of the sample was built within the ellipsometer program. For the silicon substrate the library model *Si_J AW* was used. Atop a 1.5nm thick layer of Si0₂ was assumed and implemented. For the implementation of the deposited polymer into the model a Cauchy model was used. The separate predefined library models also for layers of the specimen offer a set of variables which can be permitted to alteration in the fitting process. Regarding the Cauchy model, the exponent parameters was set to 0 and the band edge parameter to 400 nm for all measurements. Parameters alterable by the fitting routine are specified. Uncertainties are only given for the thickness values, as the remainder of the Cauchy model parameters are merely given for documentation purpose. It is however noted that their uncertainties are within reasonable limits. Obtained ellipsometry results are displayed in the following Table 5.

**Table 5: Ellipsometry fit parameters for the silicon samples. One of the samples was measured on two different points. The parameters A, B, C correspond to the parameters used in the Cauchy model.**

| **Color/Sample** | **Roughness** | **Thickness (fit)** | **A (fit)** | **B (fit)** | **C** | **k-Amplitude** |
|---|---|---|---|---|---|---|
| Red/Sample 1 | 29.10 nm | 193.1 ± 0.2 nm | 1.503 | -0.0161 | 0.00197 (fit) | 0.02596 (fit) |
| Red/Sample 2 (right) | 16.76 nm (fit) | 194.9 ± 0.3 nm | 1.496 | -0.0116 | 0.00163 (fit) | 0.02355 (fit) |
| Red/Sample 2 (left) | 19.37 nm (fit) | 210.5 ± 0.3 nm | 1.493 | -0.0081 | 0.00133 (fit) | 0.02002 (fit) |

### pH-colorimetry

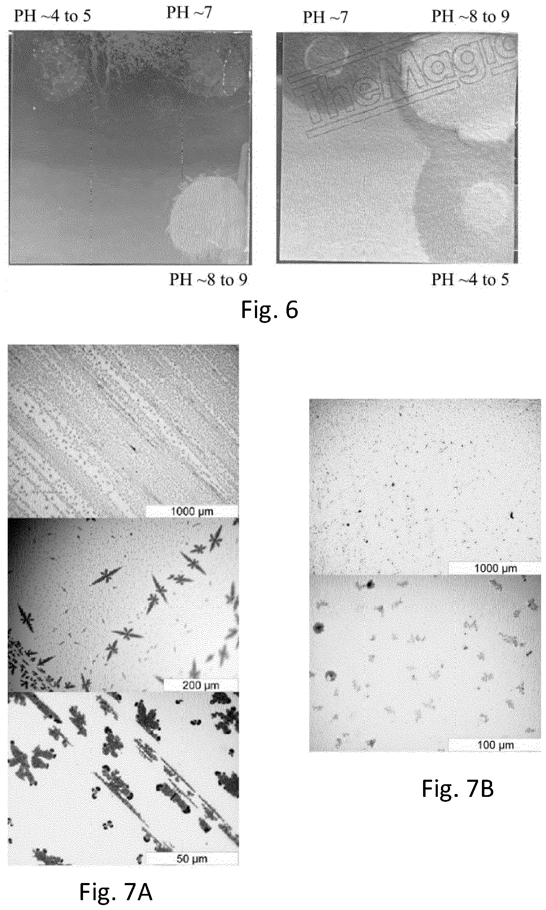

To examine the functionality of the pH indicator dye methyl red in the deposited thin film, different areas of two tattoo paper samples where subjected to drops of a mild acid at pH 4 - 5, water at pH 7 or mild base at pH 8-9. The color of the tattoo paper samples' areas where the respective drops of different pH were placed corresponded to the respective pH, i.e. at pH 4-5 the area of the deposited thin film was red and at pH 8-9 the area was yellow. The pH-colorimetry results are shown in Fig. 6, wherein the pH of the drop applied to the respective areas of the two tattoo paper samples (left and right pictures of Fig. 6) is indicated next to respective area in the left and right picture, respectively.

### Optical microscopy

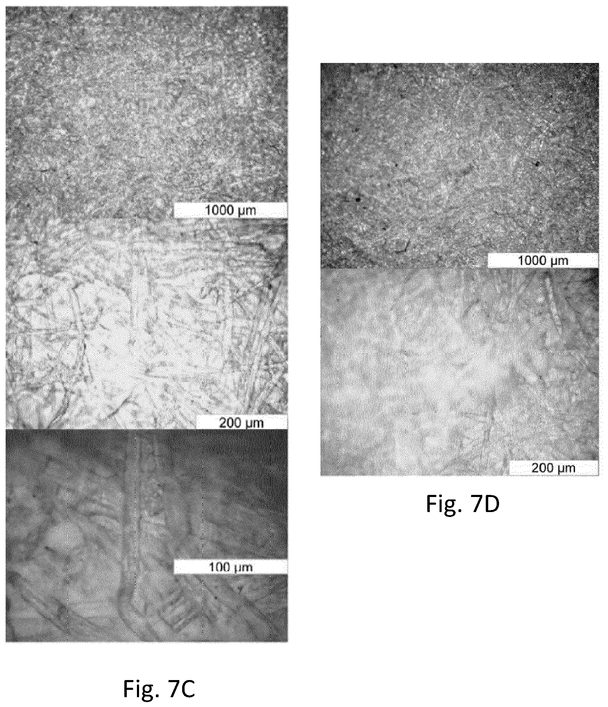

Glass, silicon and both tattoo paper samples were inspected at magnifications ranging from 50 to 1000. For the tattoo paper samples areas exposed to liquid of the pH solutions and untreated areas were targeted. Fig. 7A compares the silicon waver samples at different magnifications (50, 200, 1000). Similarly, Fig. 7B compares the corresponding glass samples (magnifications 100 and 1000) and Fig. 7C areas of the tattoo paper sample (magnifications 100, 200, 1000) that were not subjected to a pH-solution (see pH-colorimetry measurement above). Fig. 7D shows the areas of the tattoo paper sample (magnifications 200 and 1000) treated with pH-solution of pH 8-9 (see pH-colorimetry measurement above).

### UV-vis spectrophotometry

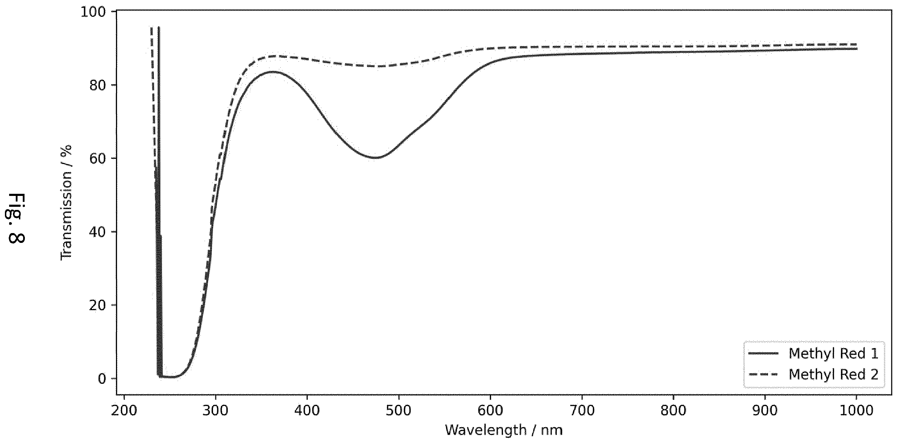

To obtain quantitative information on the amount of pH-indicator dye in the deposited polymer film, light absorption of the glass samples in dependence of wavelength was measured using a spectrophotometer. Since for the methyl red glass sample a gradient of coloring intensity was visible, two measurements were taken on either side of the sample. The measurement procedure merely consisted of placing a reference (an uncoated glass sample) and the sample to be measured in the probe chamber of the spectrophotometer. As the sample holder of the used spectrophotometer is generically intended to hold glass valves with liquids, the glass samples were fixed with tape to the sides of the sample holder. Fig. 8 shows the measured light intensity versus wavelength curves. Note: transmittances of more than 100%, recorded at wavelengths of less than 250nm, were considered to be measurement artefacts and were omitted in the plot.

### 2.4 Results and conclusion

The thickness of deposited polymer films with dye co-deposition was measured by ellipsometry. The thicknesses found to agree with the thickness estimated by laser interferometry. It was proven that a pH-indicator dye co-deposited with the hydrogel matrix via combined PVD and iCVD on a tattoo layer of a tattoo paper remained functional in the sense of changing color depending on pH-level.

The foregoing examples and described embodiments have been set forth merely to illustrate the invention and are not intended to be limiting. Since modifications of the disclosed embodiments incorporating the substance of the invention may occur to those skilled in the art, the invention should be construed to include everything within the scope of the appended claims.

## Claims

1. A temporary tattoo sensor for application to an individual's skin and for detecting physiological analytes within sweat of said individual, wherein the temporary tattoo sensor comprises:
a substrate layer configured to be placed on the skin of the individual and having a first side facing away from the skin and a second side facing the skin, when the temporary tattoo sensor is placed on the skin of the individual;
a colorimetric layer disposed on the second side of the substrate layer, wherein, when the temporary tattoo sensor is placed on the skin of the individual, the colorimetric layer is adjacent to and in direct contact with the skin;
wherein the colorimetric layer comprises a hydrogel matrix and at least one indicator dye;
wherein the hydrogel matrix is configured to accumulate sweat that segregates from the individual's skin and to act as a carrier matrix for immobilizing the at least one indicator dye; and
wherein the at least one indicator dye is configured to visibly change color upon contact with at least one physiological analyte present in the individual's sweat accumulated in the hydrogel.

2. The temporary tattoo sensor according to claim 1, further comprising
• a water-dissolvable release layer applied to the first side of the substrate layer; and
• a removable support paper applied to the water-dissolvable release layer.

3. The temporary tattoo sensor according to claim 1 or 2, **characterized in that**
the at least one indicator dye changes from a first visible color to a second visible color upon contact with the at least one physiological analyte present in the individual's sweat accumulated in the hydrogel,
or **in that**
the at least one indicator dye changes from transparent to a visible color, or vice versa, upon contact with the least one physiological analyte present in the individual's sweat accumulated in the hydrogel.

4. The temporary tattoo sensor according to any one of claims 1 to 3, **characterized in that** the at least one indicator dye is configured to visibly change color upon contact with the at least one physiological analyte which is selected from the group consisting of: pH, calcium, chloride, corticosteroids, creatinine, urea, amino acids, glucose, lactate, and combinations thereof.

5. The temporary tattoo sensor according to any one of claims 1 to 4, further comprising at least one additional indicator dye comprised in the colorimetric layer and immobilized by the hydrogel matrix, wherein the at least one additional indicator dye is configured to visibly change color upon detecting at least one further skin- or sweat-related parameter, and wherein the at least one additional indicator dye is selected from the group consisting of: a thermochromic indicator dye configured to visibly change color depending on the skin's temperature, a hydrochromic indicator dye configured to visibly change color depending on the skin's humidity or sweat rate, a photochromic indicator dye configured to visibly change color depending on the UV exposure, and combinations thereof.

6. The temporary tattoo sensor according to any one of claims 1 to 5, **characterized in that** each of the at least one indicator dye and, if present, each of the at least one additional indicator dye, is separately and visually distinguishably disposed in at least one discrete area of the colorimetric layer, wherein preferably the colorimetric layer comprises a colorimetric dye array comprising dye spots of said at least one indicator dye and at least one additional indicator dye, respectively, immobilized by the hydrogel matrix.

7. The temporary tattoo sensor according to any one of claims 1 to 6, **characterized in that** the hydrogel is selected from the group consisting of: poly-hydroxyethylacrylate (pHEMA), poly N-vinylcaprolactam /pNVCL), poly methacrylic acid (pMAA) pure and in combination with crosslinkers, such as ethylenglycoldimethacrylate (EGDMA), ethylenglycoldiacrylate (EGDA), diethylglycoldivinylether (DEGDVE), tetravinyltetramethyloctaciclosiloxane (V4D4), trivinyltrimethylesaciclosiloxane (V3D3).

8. The temporary tattoo sensor according to any one of claims 1 to 7, **characterized in that** the molecules of the at least one indicator dye and, if present, the molecules of the at least one additional indicator dye are either embedded within the hydrogel matrix or are covered by a layer of the hydrogel matrix.

9. The temporary tattoo sensor according to any one of claims 1 to 8, **characterized in that** the colorimetric layer has a thickness of 0.1 µm to 10 µm.

10. A method for manufacturing a temporary tattoo sensor according to and as defined in any one of claims 1 to 9, the method comprising depositing the colorimetric layer on the second side of the substrate layer by using solvent-based deposition techniques and/or vacuum-based deposition techniques.

11. The method according to claim 10 comprising: a step (i) of applying the at least one indicator dye dissolved in a solvent and, if present, the at least one additional indicator dye dissolved in a solvent, to the second side of the substrate layer via a solvent-based deposition technique, to form an indicator dye layer; or of applying the at least one indicator dye and, if present, the at least one additional indicator dye to the second side of the substrate layer by a vacuum-based deposition technique, to form an indicator dye layer; wherein step (i) is followed by a step (ii) of creating a layer of the hydrogel matrix on the second side of the substrate layer and on top of the deposited indicator dye layer, by means of a solution polymerization technique or by means of a vacuum-based deposition technique.

12. The method according to claim 10, the method comprising depositing the colorimetric layer on the second side of the substrate layer by using vacuum-based deposition techniques.

13. The method according to claim 12, wherein the colorimetric layer is created on the second side of the substrate layer by co-depositing (i) the at least one indicator dye and, if present, the at least one additional indicator dye, by means of Physical Vapor Deposition (PVD) and (ii) the hydrogel matrix by means of initiated Chemical Vapor Deposition (iCVD) on the second side of the substrate layer.

14. A method of monitoring the health status of an individual by measuring analytes within the individual's sweat, comprising the consecutive steps of:
• providing a temporary tattoo sensor according to any one of claims 1 to 9;
• applying the temporary tattoo sensor on the individual's skin, wherein the colorimetric layer disposed on the second side of the substrate layer is brought in direct contact with the skin, and
• monitoring the temporary tattoo sensor if a visible color change has occurred.

15. The method of claim 14 by using a temporary tattoo sensor according to any one of claims 5 to 9, wherein monitoring the health status comprises monitoring the status of a skin wound of an individual, wherein the temporary tattoo sensor is placed onto the skin wound, wherein the method comprises monitoring the temporary tattoo sensor if a visible color change with respect to at least one of the following physiological analytes and skin- or sweat-related parameters, respectively has occurred: pH, urea, glucose, amino acids, calcium, chloride, corticosteroids, creatinine, lactate, temperature, and humidity of the skin wound.
